# EUROPEAN PATENT APPLICATION

(11) **EP 4 471 061 A1**
(43) Date of publication of application: **04.12.2024**
(21) Application number: 23176616.3
(22) Date of filing: 01.06.2023
(51) Int. Cl.: C07K 16/28, A61K 47/68, C07K 16/30, A61P 35/00, A61K 39/395

(54) **ANTI-ASCT2-ANTIBODIES AND ADCS DERIVED THEREFROM**

(71) Applicant: Emergence Therapeutics AG, 47059 Duisburg (DE)
(72) Inventor: The designation of the inventor has not yet been filed
(74) Representative: Smith, Andrew George

(57) **Abstract**

The present disclosure relates to antibodies having specificity to ASCT2, antibody-drug conjugates comprising such antibodies and uses thereof.

## Description

The present invention relates to antibodies having specificity to ASCT2, antibody-drug conjugates comprising such antibodies and uses thereof.

### Background

Glutamine is the most abundant amino acid in the circulating blood (1). The glutamine dependence of cancer cells, is one of the hallmarks of tumor metabolism (2). Alanine, serine, cysteine transporter 2 (ASCT2, also reported as solute carrier family 1 member 5 or (SLC1A5)) is a Na+-coupled neutral amino acid transporter and is known as the major glutamine transporter in cancer cells (3). Upon uptake into tumor cells, glutamine is converted into intermediates of the tricarboxylic acid cycle, which provide an energy source for the various macromolecular biosynthetic pathways (4). Thus, to meet cancer cells' high energy demand, ASCT2 is often overexpressed and associated with dismal prognosis in various tumors, such as those of the non-small lung cancer (NSCLC), small cell lung cancer (SCLC), prostate cancer, colorectal cancer, head & neck cancer, and pancreatic cancer (5-7). Similarly, glutamine is important for rapidly dividing healthy cells, such as lymphocytes. Indeed ASCT2 has been shown to be important in inflammatory T-cell responses by supporting differentiation of T-helper cells to type 1 and type 2 T-helper cell subsets (8). The unique expression profile of ASCT2 in both hematological and solid tumors makes ASCT2 an interesting target for cancer therapy.

Targeting an important component of the metabolic machinery of cancer cells such as ASCT2 with an antibody or antigen-binding fragment coupled to a chemotherapeutic agent is an interesting option for the treatment of cancer.

### Description of the Invention

According to a first aspect, the present invention relates to antibodies having specificity to ASCT2 and antigen-binding fragments.

Thus, a first aspect of the invention relates to a monoclonal antibody or an antigen-binding fragment thereof which bind to ASCT2 characterized by a VH region and optionally a VL region each comprising 3 CDRs designated as CDR-H1, CDR-H2, CDR-H3, CDR-L1, CDR-L2 and CDR-L3 defining the binding specificity of the antibody or the antigen-binding antibody fragment.

Such aspect of the invention relates in particular to a monoclonal antibody or antigen-binding fragment thereof, preferably human/or humanized, which binds to ASCT2 comprising
(a) a variable heavy chain (VH) region comprising complementarity-determining regions (CDRs) CDR-H1, CDR-H2 and CDR-H3, wherein
   (i) the CDR-H1 comprises an amino acid sequence according to SEQ ID NO:1, or an amino acid sequence derived from such sequences comprising a substitution, particularly a conservative substitution of 1 or 2 amino acids,
   (ii) the CDR-H2 comprises an amino acid sequence according to SEQ ID NO:2, or an amino acid sequence derived from such sequences comprising a substitution, particularly a conservative substitution of 1 or 2 amino acids,
   (iii) the CDR-H3 comprises an amino acid sequence according to SEQ ID NO:3, or an amino acid sequence derived from such sequences comprising a substitution, particularly a conservative substitution of 1 or 2 amino acids, and optionally
(b) a variable light chain (VL) region, particularly a VL region comprising complementarity-determining regions (CDRs) CDR-L1, CDR-L2 and CDR-L3, wherein
   (i) the CDR-L1 comprises an amino acid sequence according to SEQ ID NO:5, or an amino acid sequence derived from such sequences comprising a substitution, particularly a conservative substitution of 1 or 2 amino acids,
   (ii) the CDR-L2 comprises an amino acid sequence according to SEQ ID NO:6, or an amino acid sequence derived from such sequences comprising a substitution, particularly a conservative substitution of 1 or 2 amino acids,
   (iii) the CDR-L3 comprises an amino acid sequence according to SEQ ID NO:7, or an amino acid sequence derived from such sequences comprising a substitution, particularly a conservative substitution of 1 or 2 amino acids.

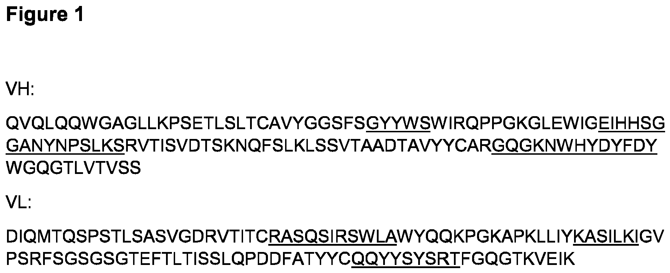

Specific amino acid sequences represented by SEQ ID NOs used herein are provided in the attached sequence listing and shown in Figure 1. The position of CDRs within a VH or VL region may be defined according to Kabat, et al., Sequences of Proteins of Immunological Interest, 5th Ed. Public Health Service, National Institutes of Health, Bethesda, MD (1991) or the IMGT numbering system, both of which are known to the person skilled in the art. If not stated otherwise, the Kabat system is used herein.

The antibodies of the invention are monoclonal antibodies (mAb) or mono-clonal antibody fragments characterized by a specific amino acid sequence. If not indicated differently, the term "monoclonal" refers to a single species, i.e. single amino acid composition of antibodies or antibody fragments.

The term "antigen-binding site" denotes the region(s) of an antibody molecule to which a ligand (e.g. the antigen, i.e. ASCT2, or antigen fragment of it) actually binds and which is derived from an antibody. An antigen-binding site of an antibody according to the invention can contain six complementarity determining regions (CDRs) which contribute in varying degrees to the affinity of the binding site for the antigen. There are three heavy chain variable domain CDRs (CDR-H1, CDR-H2 and CDR-H3) and three light chain variable domain CDRs (CDR-L1, CDR-L2 and CDR-L3). Also included within the scope of the invention are functional antigen binding sites comprised of fewer CDRs (i.e., where binding specificity is determined by three, four or five CDRs). For example, less than a complete set of 6 CDRs may be sufficient for binding. In some cases, a VH or a VL domain will be sufficient.

An "antigen-binding fragment" of an antibody refers to a molecule comprising a portion of an intact antibody that binds the antigen to which the intact antibody binds. Examples of antibody fragments include but are not limited to Fv, Fab, Fab', Fab'-SH, F(ab')2; diabodies; linear antibodies; single-chain antibody molecules (e.g. scFv); and multi-specific antibodies formed from antibody fragments. The term also encompasses a fusion protein, e.g. a fusion protein with a non-immunoglobulin peptide or polypeptide, and a conjugate with a non-proteinaceous structure, e.g. a label or a toxin. The terms "antigen-binding fragment of an antibody", "antigen-binding fragment thereof", "fragment of an antibody" or "fragment thereof" may be used interchangeably herein.

According to the present invention, a substitution of 1 or 2 amino acids in these CDR sequences is possible. In particular embodiments, a conservative amino acid substitution is preferable, i.e. a substitution of an amino acid by another amino acid with similar biochemical properties, for example a substitution of an aliphatic amino acid, e.g. Gly, Ala, Val, Leu or Ile, for another aliphatic amino acid, a basic amino acid, e.g. His, Lys or Arg, against another basic amino acid, an acidic amino acid or an amide thereof, e.g. Asp, Glu, Asn or Gln, against another acidic amino acid or an amide thereof, an aromatic amino acid, e.g. Phe, Tyr or Trp, against another aromatic amino acid, or a hydroxy or sulfur containing amino acid, e.g. Ser, Thr, Met or Cys, against another hydroxy or sulfur containing amino acid.

As used herein, the terms "binding" and "specific binding" refer to the binding of the inventive antibody or fragment thereof to an epitope of the ASCT2 antigen. The measure of the binding strength of an antibody is referred to as affinity. Methods for determining such a binding and/or affinity using in vitro assays are known to the person skilled in the art. According to the present invention, detection with flow cytometry, immuno-histochemistry and/or fluorescence are described and particularly preferred herein. The affinity of the binding of an antibody to an antigen is defined by the terms Ka (rate constant for the association of the antibody from the antibody/antigen complex), KD (dissociation constant), and K_{dis} (KD/Ka).

The inventive antibodies or the antigen-binding fragments thereof may be mono- or multivalent, i.e. it may comprise a single antigen-binding site or multiple antigen-binding sites. For example, Fab fragments have single antigen-binding site, antibodies of the IgG class or Fv or scFv fragments have two antigen-binding sites and antibodies of the IgM class have 5 antigen-binding sites. The term "antibody" also encompasses hetero-specific antibodies, e.g. hetero-bispecific antibodies, which have different antigen-binding sites, particularly antibodies, which are directed to two different epitopes on the antigen. As used herein, "epitope" is a region of an antigen that is bound by an antibody. The term "epitope" includes any polypeptide determinant capable of specific binding to an antibody.

Preferred embodiments relate to monoclonal antibodies or antigen-binding fragments thereof which bind to ASCT2 comprising

Especially preferred embodiments relate to monoclonal antibodies or antigen-binding fragments thereof which bind to ASCT2 comprising
(a) a variable heavy chain (VH) region comprising complementarity-determining regions (CDRs) CDR-H1, CDR-H2 and CDR-H3, wherein
   (i) the CDR-H1 comprises an amino acid sequence according to SEQ ID NO:1,
   (ii) the CDR-H2 comprises an amino acid sequence according to SEQ ID NO:2,
   (iii) the CDR-H3 comprises an amino acid sequence according to SEQ ID NO:3, and
(b) a variable light chain (VL) region comprising complementarity-determining regions (CDRs) CDR-L1, CDR-L2 and CDR-L3, wherein
   (i) the CDR-L1 comprises an amino acid sequence according to SEQ ID NO:5,
   (ii) the CDR-L2 comprises an amino acid sequence according to SEQ ID NO:6,
   (iii) the CDR-L3 comprises an amino acid sequence according to SEQ ID NO:7.

According to the present invention, a VH region or the CDRs thereof alone may constitute a complete antigen-binding site. In certain embodiments, the antibody comprises a VH region or the CDRs thereof as defined herein alone.

Thus, a further aspect of the invention relates to a monoclonal antibody or antigen-binding fragment thereof binding to ASCT2 comprising
(a) a VH region comprising an amino acid sequence according to SEQ ID NO:4, or an amino acid sequence having an identity thereof of at least 85%, at least 90%, at least 95% or at least 99%, and optionally
(b) a VL region, particularly a VL region comprising an amino acid sequence according to SEQ ID NO:8, or an amino sequence having an identity thereof of at least 85%, at least 90%, at least 95% or at least 99%.

Preferred are monoclonal antibodies or antigen-binding fragments thereof binding to ASCT2 comprising
(a) a VH region comprising an amino acid sequence according to SEQ ID NO:4, and
(b) a VL region comprising an amino acid sequence according to SEQ ID NO:8.

Especially preferred is the antibody 17c10 (Figure 1).

"Percent (%) amino acid sequence identity" with respect to a peptide or polypeptide sequence is defined as the percentage of amino acid residues in a candidate sequence that are identical with the amino acid residues in the specific peptide or polypeptide sequence, after aligning the sequences and introducing gaps, if necessary, to achieve the maximum percent sequence identity, Alignment for purposes of determining percent amino acid sequence identity can be achieved in various ways that are within the skill in the art, for instance, using publicly available computer software such as BLAST.

In certain embodiments, antibody of the conjugate of the invention may be a chimeric antibody, a multispecific antibody, in particular a bispecific antibody, a human antibody, a humanized antibody, or an antigen-binding fragment thereof.

According to the present invention, a "chimeric" antibody refers to an antibody in which a portion of the heavy and/or light chain is derived from a particular source or species, while the remainder of the heavy and/or light chain is derived from a different source or species.

"Multispecific antibodies" bind two or more different epitopes. The epitopes may be on the same or different antigens. A preferred example of a multispecific antibody is a "bispecific antibody" which binds two different epitopes.

In preferred embodiments, the antibody of the invention is a human or humanized antibody.

The term "humanized antibody" or "humanized version of an antibody" refers to antibodies for which both heavy and light chains are humanized as a result of antibody engineering. A humanized chain is typically a chain in which the V-region amino acid sequence has been changed so that, analyzed as a whole, is closer in homology to a human germline sequence than to the germline sequence of the species of origin. For example, a murine CDR may be grafted into the framework region of a human antibody to prepare the "humanized antibody." See, e.g., Riechmann, L., et al., Nature 332 (1988) 323-327; and Neuberger, M. S., et al., Nature 314 (1985) 268-270. Other forms of humanized antibodies encompassed by the present invention are those in which the constant region has been additionally modified or changed from that of the original antibody to generate the properties according to the invention. Humanization assessment is based on the resulting amino acid sequence and not on the methodology per se.

The term "human antibody", as used herein, is intended to include antibodies having variable and constant regions derived from human germ line immunoglobulin sequences. Human antibodies are well-known in the state of the art (van Dijk, M. A., and van de Winkel, J. G., Curr. Opin. Chem. Biol. 5 (2001) 368-374). Human antibodies can also be produced in transgenic animals (e.g., mice) that are capable, upon immunization, of producing a full repertoire or a selection of human antibodies in the absence of endogenous immunoglobulin production.

Humanized or human antibodies may be also defined by their VH and/or VL regions.

The inventive antibodies invention may be of any suitable class. The term "class" refers to the type of constant domain or constant region possessed by its heavy chain. As used herein, "constant domain" or "constant region" denotes the sum of the domains of an antibody other than the variable region. The constant region is not directly involved in binding of an antigen but exhibits various effector functions. The antibody may be of any of the five major classes of antibodies, particularly of the five major classes of human antibodies: IgA, IgD, IgE, IgG, and IgM, or any subclass thereof (isotype), e.g., IgG1, IgG2, IgG3, IgG4, lgA1, and IgA2. The heavy chain constant domains that correspond to the different classes of immunoglobulins are called a, δ, ε, γ and µ, respectively. According to the present invention, an antibody of the class, particularly of the human class IgG, IgA or IgM or a fragment thereof is particularly suitable.

According to a preferred embodiment, an antibody of the conjugate of the invention is selected from class IgG, particularly from the class of human IgG, e.g., of subclass IgG1, IgG2, IgG3 of IgG4, of class IgM, of class IgA or an antigen-binding fragment thereof.

In certain embodiments, the antibody comprises a constant domain, particularly a heavy chain constant domain, more particularly a heavy chain constant domain of the class IgG, particularly of the class human IgG, e.g., of subclass IgG1, IgG2, IgG3 of IgG4, of class IgM, of class IgA, which has a reduced effector function compared to a wild-type sequence of the same subclass, e.g., which has a reduced binding to the Fc receptor. Examples of heavy chain constant domains with reduced effector functions may contain at least one of the mutations D265C, L234A, L234F, L235A, L235E, P331S, L234Q and L235F of human IgG, e.g., IgG1 or IgG4 sequences. L234F, L325E and P331S are preferred and in particular a combination of all three mutations.

According to a further embodiment, inventive antibodies or antigen-binding fragments thereof comprise a labeling group and/or an effector group being coupled to the antibody or antigen-binding fragment. The labeling group may be, for example, a dye, a paramagnetic, radioactive or fluorogenic group that is detectable upon imaging. A preferred effector group is a therapeutic group, in particular a cytotoxic agent, such as chemotherapeutically active agents, drugs, antiinflammatory agents, radioactive isotopes, toxins such as topoisomerase poisons, enzymes and fragments thereof such as nucleolytic enzymes, growth inhibitory agents, antibiotics as wells as all suitable anticancer and antitumor agents known to the person skilled in the art. Especially preferred is the topoisomerase poison Camptothecin and derivatives and/or structural analogues thereof like Exatecan as well as derivatives thereof like Deruxtecan.

Further, the present invention relates to a nucleic acid molecule, e.g. a DNA molecule, encoding an antibody VH region, or an antibody VL region, or encoding a complete antibody or an antibody fragment as indicated above, a vector or vector system, i.e. a plurality of vectors, comprising said nucleic acid molecule(s) as indicated above, preferably in operative linkage with an expression control sequence, particularly with a heterologous expression control sequence.

Furthermore, the invention relates to a cell comprising a nucleic acid molecule or a vector or vector system as described above. The term "vector," as used herein, refers to a nucleic acid molecule capable of propagating another nucleic acid to which it is linked. According to a preferred embodiment, the vector is an expression vector. An "expression vector" is a vector are capable of directing the expression of nucleic acids to which they are operatively linked. Vectors, in particular expression vectors, for the recombinant production of antibodies are well known in the art.

The cell may be a known host cell for producing antibodies or antibody fragments, e.g. a prokaryotic cell such as an E. coli cell, a yeast cell, an insect cell or a mammalian cell, e.g. a CHO cell or a hybridoma cell.

### ANTI-ASCT2 ANTIBODY DRUG CONJUGATES

A further aspect of the present invention relates to an antibody-drug conjugate (ADC) comprising a monoclonal antibody or an antigen-binding fragment binding to ASCT2 as herein described and wherein the drug is preferably a topoisomerase I inhibitor, particularly exatecan or deruxtecan.

The low expression of ASCT2 in normal tissues as well as its strong selective expression in cancer cells make of ASCT2 a reliable candidate for ADC development.

### TOPOISOMERASE I INHIBITORS

The drug of the antibody conjugate of the invention is preferably a topoisomerase I inhibitor. A topoisomerase I inhibitor is a compound, which is capable of forming a ternary complex with topoisomerase I and DNA, thereby preventing DNA re-ligation, and introducing DNA strand breaks in the cellular genome. The topoisomerase I inhibitor may be e.g., selected from camptothecin or analogs thereof, indenoisoquinolines and indolocarbazoles.

In a particular embodiment, the topoisomerase-I-inhibitor is camptothecin or an analog thereof, i.e. a compound comprising the pentacyclic basic structure of camptothecin and modified substituents optionally resulting in the presence of a further ring. Specific examples are camptothecin, topotecan, irinotecan, SN-38, belotecan, exatecan including derivatives thereof such as deruxtecan, lurtotecan or atiratecan. Further suitable camptothecin derivatives are described in Med Res Rev. 2015 Jul; 35(4): 753-789, which are incorporated by reference.

In a particular embodiment, the topoisomerase I inhibitor is exatecan:

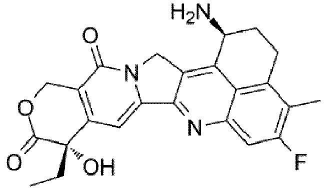

In certain embodiments, exatecan is conjugated to a ASCT2-binding agent, e.g., an antibody or antigen-binding fragment thereof, via its NH₂ group.

In further embodiments, the topoisomerase I inhibitor is a camptothecin derivative described in EP 22 177 182.2, the content of which is herein incorporated by reference, represented by the following formula 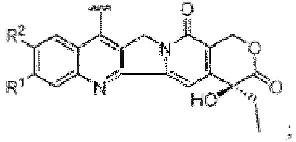 wherein
- R¹: is -F, -CH₃, or -CF₃, preferably -F,
- R²: is -H, -F, -OR³, -SR³, -S(O)R⁴, -S(O)₂R⁴, C₁-C₆ alkyl, or C₁-C₆ fluoroalkyl, preferably -F or methyl; or R¹ and R² taken together with the carbon atoms to which they are attached form a methylene dioxy or a difluoromethylene dioxy ring;
- R³: is H or C₁-C₆ alkyl; and
- R⁴: is C₁-C₆ alkyl.

In still further embodiments, the topoisomerase I inhibitor is a camptothecin derivative described in EP 22 186 381.4, the content of which is herein incorporated by reference, represented by the following formulae 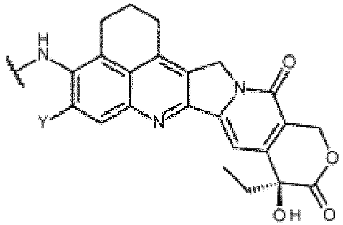 wherein
- Y: is -H, hydroxyl, C₁-C₆ alkyl, C₂-C₆ alkenyl, C₂-C₆ alkynyl, C₁-C₆ alkoxy, C₁-C₆ aminoalkoxy, C₁-C₆ alkylthio, C₁-C₆ hydroxyalkyl, C₁-C₆ haloalkyl, C₁-C₆ cyanoalkyl, C₁-C₆ nitroalkyl, halogen, nitro, cyano, or mercapto, preferably -H or -F,
or 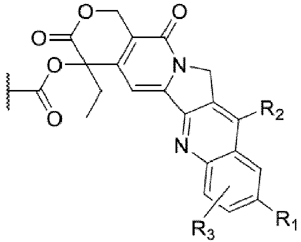
wherein
- R¹: is -CH₃, optionally substituted by halogen, preferably -CF₂H,
- R²: is -H, or C₁-C₆ alkyl, and
- R³: is H, amino, or C₁-C₆ alkyl.

Beside topoisomerase I inhibitors, for example, topoisomerase II inhibitors, microtubulin inhibitors like monomethyl auristatin A MMAE, monomethyl auristatin F MMAF or maytansine, DNA binders like calicheamicin and derivatives thereof, glucocorticoid receptor modulators (GRM) like dexamethasone or budesonide as well as derivatives thereof but also alternatives to small molecule payloads such as siRNAs etc. may be used.

### CONJUGATION

The topoisomerase-I-inhibitor, particularly exatecan, is covalently conjugated to the anti-ASCT2 antibody or antigen-binding fragment thereof.

In principle, the topoisomerase-I-inhibitor, e.g. exatecan, may be conjugated to any suitable position of the anti-ASCT2 antibody or antigen-binding fragment thereof, particularly to any position, which does not abolish the binding of the anti-ASCT2 antibody or antigen-binding fragment thereof. For example, the topoisomerase-I-inhibitor, e.g., exatecan, may be conjugated to a reactive amino acid residue on an antibody, e.g., an amino acid residue having a side chain comprising an amino, hydroxy or thiol group, or a reactive group in the antibody glycan structure. In particular embodiments, the topoisomerase-I-inhibitor, e.g., exatecan, is conjugated to a reactive thiol group in the side chain of an accessible cysteine residue on an antibody.

In certain embodiments, the antibody drug conjugate comprises has a molar drug-binding agent (e.g., antibody/antibody fragment) ratio (DAR) of greater than 1, i.e., more than one drug molecule is attached to a molecule of the ASCT2 binding agent, e.g., an antibody/antibody fragment. Typically, the conjugate has a DAR of about 2:1 to about 16:1, more preferably of about 2:1 to about 8:1, particularly of about 4:1. The DAR may be calculated from a statistical distribution according to known methods.

In certain embodiments, the topoisomerase-I-inhibitor is conjugated to the anti-ASCT2 antibody or antigen-binding fragment thereof via a linker. In certain embodiments, the linker is a cleavable linker, i.e., a linker cleavable under physiological conditions, e.g., by physiological enzymes. Specific examples of cleavable linkers are peptide-based linkers, which may be subject to cleavage by a protease, or glycoside-based linkers, which may be subject to cleavage by a glycosidase. A preferred example of an enzymatically cleavable linker is a valine-citrulline linker or a glutamic acid-valine-citrulline linker. Such linkers are well known to the person skilled in the art and for example described in Anami at al, Nature Communications 9, Article number: 2512 (2018).

In certain embodiments, the linker is a hydrophilic polysarcosine linker e.g., as described by Conilh et at. "Exatecan antibody drug conjugates based on a hydrophilic polysarcosine drug-linker platform" (Pharmaceuticals 14 (2021), 247). Further preferred linkers include linkers comprising at least one ethylene glycol unit, e.g., 1, 2, 3, 4, 5, 6, 7, 8, 9, or 10 or more ethylene glycol units, e.g., the linker of the antibody-drug conjugate MED17247, an mcc-triazole spacer-PEG7-x-Lys-PABC glycol linker from Trodelvy^{®}, Further preferred linkers include oligopeptide, particularly di- to decapeptide, e.g., tetrapeptide sequences such as glycine-glycine-phenylalanine-glycine from Enhertu^{®}. Further preferred linkers include highly polar spacers such as an acyl group, carbamoyl group and/or sulfamide group added to at least at least one ethylene glycol unit, e.g., 1, 2, 3, 4, 5, 6, 7, 8, 9, or 10 or more ethylene glycol units.

In certain embodiments, the linker is a hydrophilic polysarcosine linker comprising, e.g., up to 15 sarcosine units, and at least one ethylene glycol unit, e.g., 1, 2, 3, 4, 5, 6, 7, 8, 9, or 10 or more ethylene glycol units, wherein the linker is subject to cleavage by a glycosidase, and particularly subject to cleavage by a glucuronidase.

In certain embodiments, the linker is a hydrophilic polysarcosine linker comprising, e.g., about 8-12 sarcosine units, and at least one ethylene glycol unit, e.g., up to 10 ethylene glycol units, and wherein the linker is subject to cleavage by a glycosidase, and particularly subject to cleavage by a glucuronidase.

In certain embodiments, the linker is a hydrophilic polysarcosine linker comprising 10 sarcosine units, and 2 ethylene glycol units, and wherein the linker is subject to cleavage by a glucuronidase.

In certain embodiments, a linker-drug conjugate comprises:
(i) a hydrophilic polysarcosine linker comprising, e.g., about 8-12 sarcosine units, and at least one ethylene glycol unit, e.g., 1, 2, 3, 4, 5, 6, 7, 8, 9, or 10 or more ethylene glycol units, wherein the linker is subject to cleavage by a glycosidase, and particularly subject to cleavage by a glucuronidase, and wherein the linker comprises a thiol-reactive group, e.g., a maleimide group capable of reacting with cysteine residues on an antibody or antigen-binding fragment thereof, and
(ii) a topoisomerase I inhibitor, particularly exatecan, covalently attached to the linker.

In particular embodiments, the linker-drug conjugate comprises:
(i) a hydrophilic polysarcosine linker comprising 10 sarcosine units, and 2 ethylene glycol units, wherein the linker is subject to cleavage by a glycosidase, and particularly subject to cleavage by a glucuronidase, and wherein the linker comprises a maleimide group capable of reacting with cysteine residues on an antibody or antigen-binding fragment thereof, and
(ii) a topoisomerase I inhibitor, particularly exatecan covalently attached to the linker.

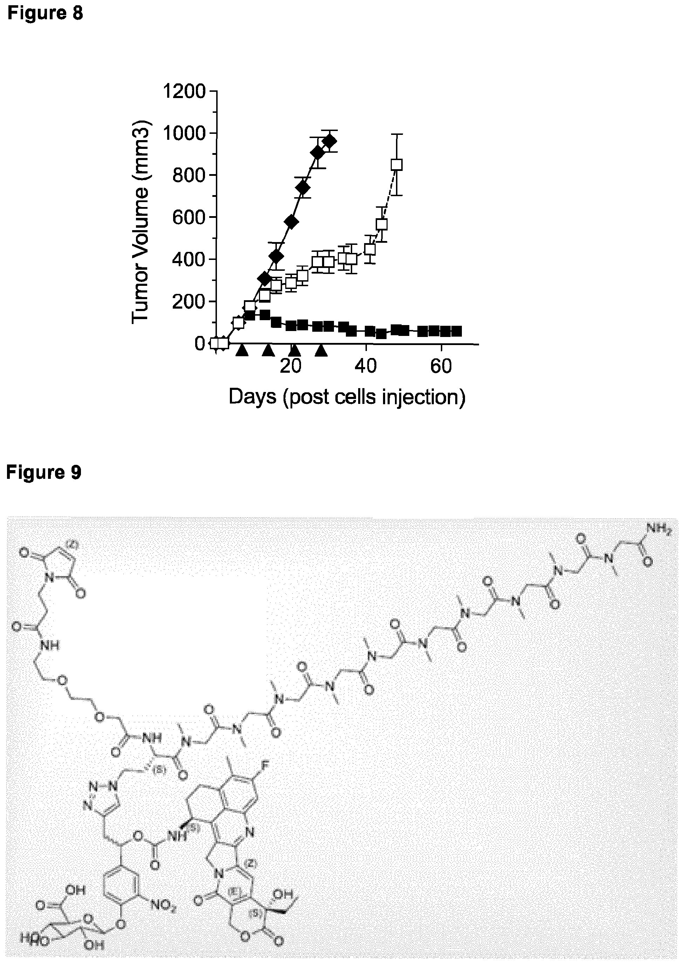

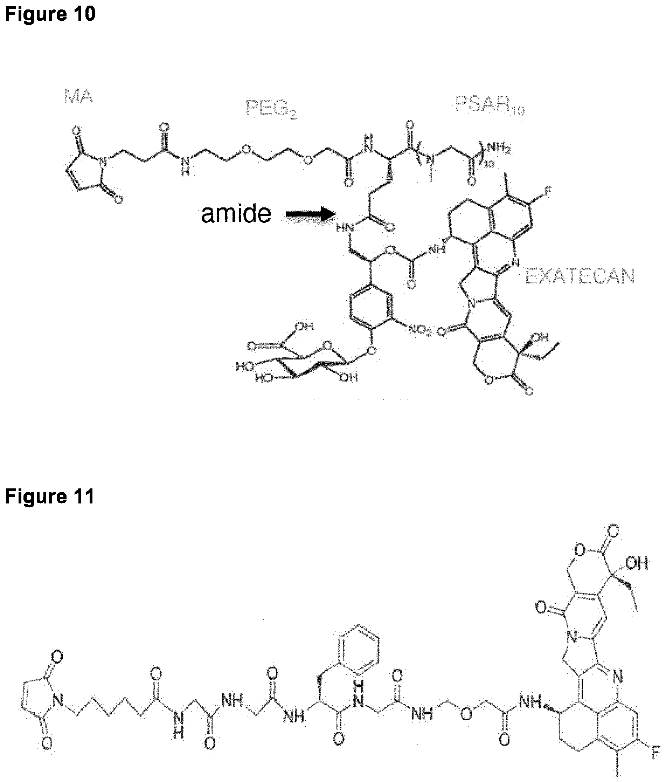

Especially preferred linker-drug conjugates according to this embodiment are shown in Figures 9 and 10.

Figure 11 illustrates another preferred linker-drug conjugate for use in the present invention.

The ADC may be prepared by any known methods.

The antibody or antigen-binding fragment thereof may be reacted with a linker-drug conjugate to obtain the conjugate. The linker-drug conjugate comprises a drug molecule, e.g., having attached thereto a suitable linker, wherein the linker comprises a reactive group capable of reacting with desired attachment positions on the binding agent, e.g., the antibody or antigen-binding fragment thereof. For attachment to cysteine residues, the linker-drug conjugate comprises a thiol-reactive group, e.g., a maleimide group. The use of maleimide chemistry for providing ADCs is well known to the person skilled in the art. A recent review on corresponding cysteine bases coupling is You et al., Biconjugate Chem. 2021, 32, 8, 1525-1534.

Another well-known method for attaching a linker-drug conjugate to a given antibody is based on consecutive enzymatic glycan trimming and tagging of each N-glycan with precisely one azide at an asparagine, in particular asparagine-297, of the antibody. The azide provides a unique anchor point introduced for copper-free click conjugation of the linker-drug conjugate, such as by strain-promoted azide-alkyne cycloaddition. Of course, any other kind of click chemistry known to the person skilled person known for site-specific antibody-drug conjugation can be used, such as hydrazine-iso-Pictet Spengler, trapped-Knoevenagel, tandem Knoevenagel condensation Michael addition, copper-catalyzed azide-alkylene cycloaddition, inverse-electron-demand Diels Alder cycloaddition or Diazaborine (DAB). A recent review on click chemistry conjugations was published by Ohio and Bane, Methods Mol Biol. 2020, 2078:83-97. Of course, also enzymatic conjugation, for example by the use of bacterial transglutaminase (BTG), is also possible.

The anti-ASCT2 antibody or antigen-binding fragment thereof may be produced by known methods, e.g., by chemical synthesis methods, e.g., peptide solid synthesis, and/or by biological methods, e.g., production in a recombinant host cell or organism.

For example, the antibody or antigen-binding fragment thereof may be produced in a suitable host cell comprising a nucleic acid molecule, e.g., a DNA molecule, encoding an antibody VH region, or an antibody VL region, or encoding a complete antibody or an antibody fragment, or a vector or vector system, i.e., a plurality of vectors, comprising said nucleic acid molecule(s), preferably in operative linkage with an expression control sequence, particularly with a heterologous expression control sequence. The host cell may be any known host cell for producing antibodies or antibody fragments, e.g., a prokaryotic cell such as an E. coli cell, a yeast cell, an insect cell, or a mammalian cell, e.g., a CHO cell or a hybridoma cell.

The linker-drug conjugate is particularly suitable for attachment to an anti-ASCT2 antibody as described herein. It should be noted, however, that the linker-drug conjugate is also suitable for attachment to any ASCT2-binding agent.

### PHARMACEUTICAL COMPOSITIONS

Further disclosed is a pharmaceutical composition comprising an anti-ASCT2 antibody or an ADC as herein described, e.g., a monoclonal antibody or an antigen-binding fragment thereof and a drug which is preferably a topoisomerase I inhibitor, particularly exatecan, and a pharmaceutically acceptable carrier and/or excipient.

Examples of suitable carriers and excipients for formulating antibody drug conjugates include saline and aqueous buffer solutions and are well known in the art. Typically, the pharmaceutical composition is adapted for parenteral administration, e.g., for subcutaneous, intramuscular, or intravenous injection or by infusion. In further embodiments, the pharmaceutical composition is adapted for local administration, e.g., for intravesical instillation into the bladder.

Depending on the stage and the severity of the disorder, the pharmaceutical composition may be administered once or several times in a therapeutically effective dose to a subject in need thereof, particularly to a human subject. For example, it may be administered once or several times daily, each second day, two times weekly or weekly for a suitable period, e.g., of at least one week, or at least one month.

### MEDICAL APPLICATIONS

The conjugate or pharmaceutical composition as described above is used in human medicine for administration to a subject suffering from an anti-ASCT2 associated disorder.

Expression of ASCT2 was shown to vary widely among tumor types. Compared to healthy tissue, ASCT2 is significantly upregulated in non-small lung cancer (NSCLC), small cell lung cancer (SCLC), prostate cancer, colorectal cancer, head & neck cancer, and pancreatic cancer. Thus, in certain embodiments, the anti-ASCT2 associated disorder is ASCT2 positive cancer, in particular a cancer characterized by strong ASCT2 expression. However, the cancer may be any kind of cancer, wherein the term "cancer" is used herein to refer to proliferative diseases. Particularly, the cancer is an ASCT2-positive cancer selected from lung, prostate, head and neck, pancreatic, and/or colorectal cancer, more particularly an ASCT2-positive cancer selected from lung, head and neck, and/or colorectal cancer.

In therapeutic applications, the active agent is administered in an effective amount to a subject, particularly to a mammalian subject, and more particularly to a human subject. The dose will depend on the specific type of agent, e.g., type of antibody or antibody fragment, the type of disease, and the mode of administration, e.g., locally, or systemically.

The antibody or antibody drug conjugate may be administered alone or together with a further active agent, which may be selected from chemotherapeutic agents, e.g., anti-metabolites, alkylating agents, intercalating agents, or anti-mitotic agents), inhibitors of specific kinases e.g., tyrosine kinase inhibitors, serine/threonine kinase inhibitors or phosphoinositide kinase inhibitors, immunotherapeutic compounds, e.g., immune checkpoint inhibitors, CAR-T cells, therapeutic vaccines, or oncolytic viruses.

The inventive antibodies and fragments thereof may be also used in diagnostics, for example being coupled to a labeling group as described above. Thus, according to a further aspect of the invention, the inventive antibody or antigen-binding fragment thereof is for use in medicine, particularly for therapeutic or diagnostic applications including in vitro and in vivo diagnostic applications.

Further, the present invention is explained in more detail by the following Tables, Figures and Examples.

### Figures

- **Figure 1:**: VH and VL sequence of antibody 17c10. CDR sequences are underlined (Kabat format)

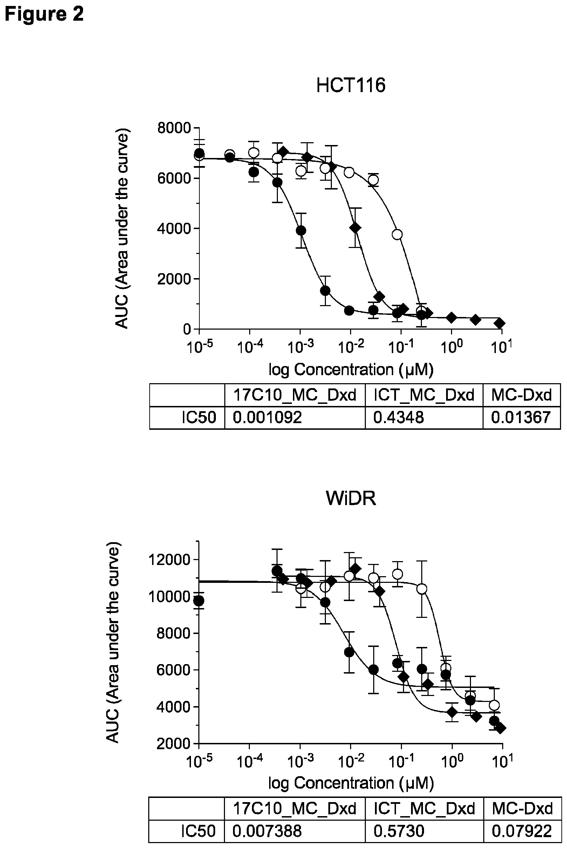

- **Figure 2:**: 17c10_MC-Dxd cytotoxic effect on HCT116 and WiDr cell lines determined with Incucyte read-out.

HCT116 (first panel) or WiDr cells (second panel) were incubated as described in Example 3 and Table 1 with a dose range of 17c10_MC-Dxd (black circles) and ICT_MC-Dxd (open circles) or linker payload MC-Dxd alone (black diamonds). Cytotoxic effect was observed on HCT116 and WiDr with the 17c10_MC-Dxd ADC. Non-specific cytotoxicity was only observed at the highest concentrations with the isotype control (ICT_MC-Dxd). Better cytotoxic effect (lower IC₅₀) was observed with 17c10_MC-Dxd ADC compared to free MC-Dxd drug-linker on the two cell lines.

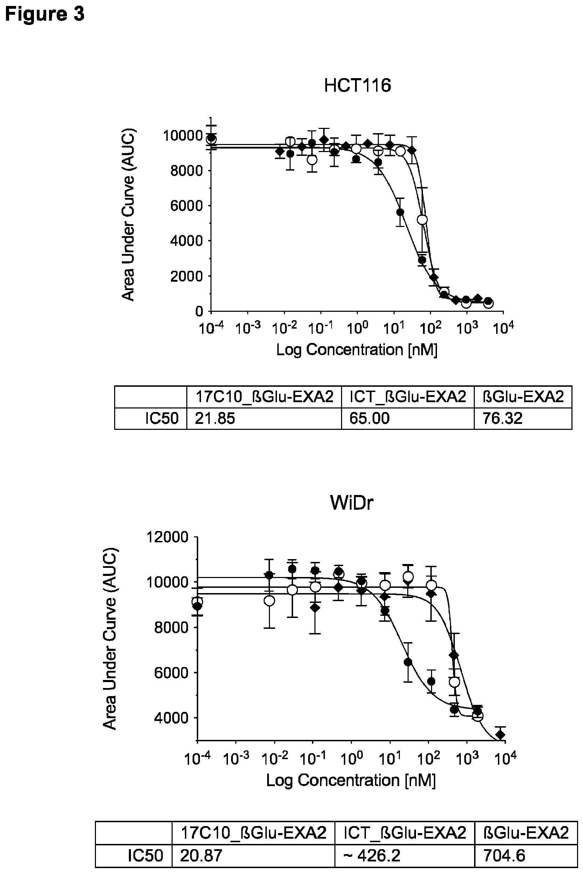

- **Figure 3:**: 17c10_βGlu-EXA2 cytotoxic effect on HCT116 and WiDr cell lines determined with Incucyte read-out.

HCT116 (first panel) or WiDr cells (second panel) were incubated as described in Example 3 and Table 1 with a dose range of 17c10_βGlu-EXA2 (black circles) and ICT_ βGlu-EXA2 (open circles) or linker payload MC- βGlu-EXA2 (black diamonds). Cytotoxic effect was observed on HCT116 and WiDr with the 17c10_βGlu-EXA2. Non-specific cytotoxicity was only observed at the highest concentrations with the isotype control (ICT_ βGlu-EXA2). Better cytotoxic effect was observed with 17c10_βGlu-EXA2 ADC compared to free βGlu-EXA2 drug-linker on the two cell lines.

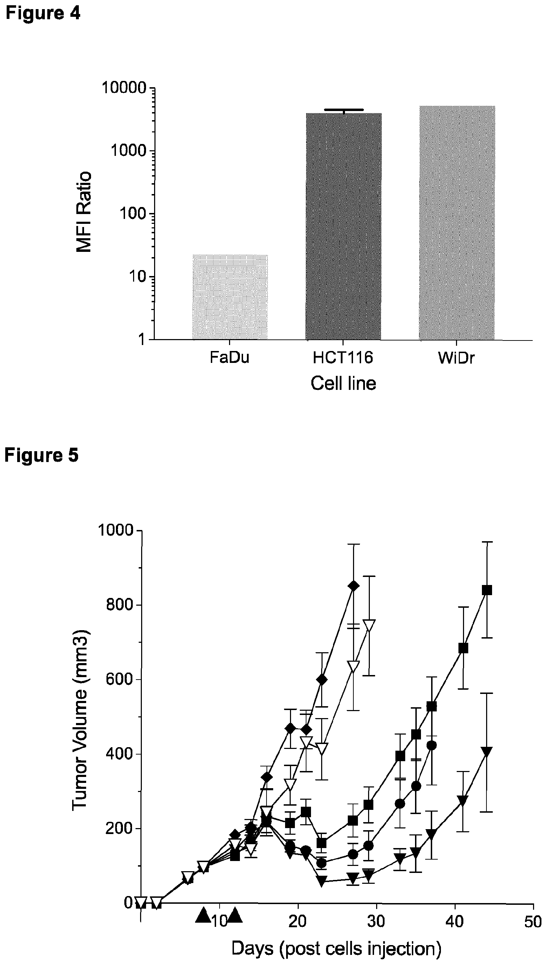

- **Figure 4:**: Expression of human ASCT2 on various cells lines as determined by flow cytometry.

Histograms showing the ratios of flow cytometry signals observed after various cell lines incubation with primary antibody 17c10 over its isotypic control. High ratios indicate high level of human ASCT2 expression at the surface of tested cell lines.
- **Figure 5:**: Treatment of WiDr grafted NMRI nude mice with 17c10_MC-Dxd

NMRI mice (n=10/group) were xenografted with WiDr cells embedded in Matrigel. Two different ADC were tested: Isotypic Control, ICT- and 17C10_MC-Dxd. Treatment of mice (2 intravenous injection, black arrowhead) started when tumors reached approximately 100 mm³. 17c10_MC-Dxd was evaluated at 3 doses (2, 5 and 10 mg/kg; black squares, circles and inverted triangles, respectively), ICT_MC-Dxd was given at 10 mg/kg (open inverted triangles). Control animals were treated with ADC diluent (black diamonds). Tumor volumes were monitored with a caliper twice a week thereafter and values were reported with the following formula (Length × Width² × (3.14/6). A dose-dependent tumor regression and control was observed in comparison to controls. Recurrence was observed in all cases.

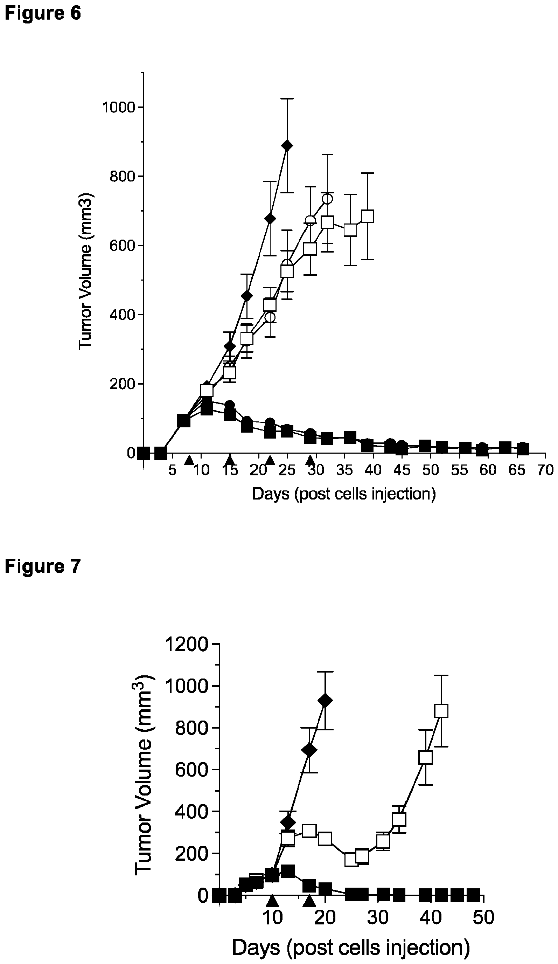

- **Figure 6:**: Treatment of WiDr grafted NMRI nude mice with 17c10_βGlu-EXA1 or 17c10_βGlu-EXA2

NMRI mice (n=10/group) were xenografted with WiDr cells embedded in Matrigel. Four different ADC were tested: ICT_ and 17c10_βGlu-EXA1, ICT_ and 17c10_βGlu-EXA2. Treatment of mice (4 intravenous injection, black arrowhead) started when tumors reached approximately 100 mm³. ICT_ and 17c10_βGlu-EXA1 were evaluated at 10 mg/kg (open and black squares, respectively), ICT_ and 17c10_βGlu-EXA2 were tested at 5 mg/kg (open and black circles, respectively). Control animals were treated with ADC diluent (black diamonds). Tumor volumes were monitored with a caliper twice a week thereafter and values were reported with the following formula (Length × Width² × (3.14/6). Almost complete tumor regression and long-term tumor control was observed with both 17c10_βGlu-EXA1 and EXA2 ADC in comparison to respective controls and buffer-treated animals.
- **Figure 7:**: Treatment of FaDu grafted NMRI nude mice with 17c10_βGlu-EXA1

NMRI mice (n=10/group) were xenografted with FaDu cells. Two different ADC were tested: ICT_ and 17c10_βGlu-EXA1. Treatment of mice (2 intravenous injection, black arrowhead) started when tumors reached approximately 100 mm³. ICT_ and 17c10_βGlu-EXA1 were evaluated at 10 mg/kg (open and black squares, respectively). Control animals were treated with ADC diluent (black diamonds). Tumor volumes were monitored with a caliper twice a week thereafter and values were reported with the following formula (Length × Width² × (3.14/6). Complete tumor regression and long-term tumor control was observed with 17c10_βGlu-EXA1 ADC in comparison to isotypic controls ADC and buffer-treated animals. Of note, the isotypic control ADC had a temporary effect on tumor growth, but tumors relapsed after end of treatment.
- **Figure 8:**: Treatment of HCT116 grafted NMRI nude mice with 17c10_βGlu-EXA1

NMRI mice (n=10/group) were xenografted with HCT116 cells. Two different ADC were tested: ICT_ and 17c10_βGlu-EXA1. Treatment of mice (4 intravenous injection, black arrowhead) started when tumors reached approximately 100 mm³. ICT_ and 17c10_βGlu-EXA1 were evaluated at 10 mg/kg (open and black squares, respectively). Control animals were treated with ADC diluent (black diamonds). Tumor volumes were monitored with a caliper twice a week thereafter and values were reported with the following formula (Length × Width² × (3.14/6). Long-term tumor control was observed with 17c10_βGlu-EXA1 ADC in comparison to isotypic controls ADC and buffer-treated animals. Of note, the isotypic control ADC had a temporary effect on tumor growth, but tumors relapsed after end of treatment.
- **Figure 9:**: linker-drug conjugate

The conjugate comprises a hydrophilic polysarcosine linker comprising a maleimide group, 2 etylene glycol units, 10 sarcosine units and a triazole ring, and a topoisomerase I inhibitor, particularly exatecan covalently attached to the linker. The linker is subject to cleavage by a glucuronidase.
- **Figure 10:**: linker-drug conjugate

The conjugate comprises a hydrophilic polysarcosine linker comprising a maleimide group, 2 etylene glycol units and 10 sarcosine units, and a topoisomerase I inhibitor, particularly exatecan covalently attached to the linker. The linker is subject to cleavage by a glucuronidase.
Chemical Formula: C₈₇H₁₁₂FN₁₉O₃₃
Exact Mass: 1969.76540
Molecular Weight: 1970.95140
   - **Figure 11:**: Maleimidocaproyl-GGFG-Exatecan

The linker-drug conjugate comprises a protease-cleavable linker comprising a maleimide group, an oligopeptide, particularly glycine-glycine-phenylalanine-glycine, and a topoisomerase I inhibitor, particularly exatecan covalently attached to the linker.

### Examples

### Example 1 - Antibody production

The variable VH and VK domains of interest were cloned by In-Fusion (Clontech) into transient expression vectors after PCR amplification with Clone Amp HiFi polymerase (Takara), purification using Nucleospin Gel and PCR Clean-up (Macherey Nagel). Expression vectors were then sequenced by MWG Eurofins and, following sequences validation, midi-preparation of the corresponding DNA was performed for further transfections. The human IgG1 format used is a Fc silent format with L234F, L235E and P331S mutations.

Light and heavy chains vectors were co-transfected at a 1:1.2 ratio into HEK293 cells seeded at 2.9×10⁶ cells/mL using a 1:2 DNA: transfection reagent ratio. Feeds were added 18h post-transfection and cells were kept in culture for 7 days. Supernatants were then harvested, clarified and antibodies' productivities were evaluated on Octet RED96 (PALL forte Bio) Platform.

Antibodies were purified from supernatants after overnight incubation at +4°c with a MabSelect PrismA resin (GE Healthcare, #17-5498-99) using a 0.5 M Glycine, 3 M NaCl pH 8.9 binding buffer. Samples were eluted using a 0.5 M Arginine pH 3.8 elution buffer and immediately neutralized with 1 M Tris-HCl pH 9 (10% V/V). They were then further purified by Size Exclusion Chromatography (SEC) using Superdex200 26/600 column (GE Healthcare #28-9893-36) and dialyzed against PBS 1X, pH 7.4 (Slide-A-Lyzer-G2 10KDa MWCO dialysis devices, Thermo Scientific #87731/2). They were finally concentrated on AmiconUltra 30KDa (Millipore #UFC903024) and filtrated on 0.22 µM filter (Pall #PN4905).

### Example 2 - Conjugation procedure

For MC-Dxd conjugation, Isotypic control and anti-huASCT2 17c10 antibodies (PBS1X, 1mM EDTA, pH7.4 at 12.48 and 9.21 mg/mL, respectively) were reduced by adding 14 antibody molar equivalents of TCEP (Tris(2-carboxyethyl)phosphine) for 2h at +37°C with gentle stirring. Buffer exchange into 100 mM potassium phosphate, 1 mM EDTA, pH7.4 was then performed by diafiltration using an Amicon 50 kDa centrifugal filter for less than 90min to prevent reoxidation. Antibody concentration after buffer exchange was typically >9 mg/mL.

Conjugation was performed by addition of 10.5 antibody molar equivalents of Maleimidocaproyl-GGFG-Dx8951 (MC-Dxd) drug-linker and incubation for 1h at room temperature under gentle stirring. At this step, the concentration of the ADCs was around 7 mg/mL. DAR evaluation was performed by LCMS in DTT reduced conditions on an Aeris 3.6 µm Widepore XB-C18 column (200 Å, 150 × 2.1 mm, #00F-4482-AN, Phenomenex) and was estimated to be 7.97 for isotypic control and 7.72 for anti-huASCT2 antibody. Quenching was then performed using 4 drug molar equivalents of N-acetyl cysteine (NAC) for 30min at RT.

Antibody-drug conjugate (isotypic control) was then diafiltrated on Amicon Ultra-15 centrifugal filter (50 KDa MWCO, ref#905024, Millipore) to exchange buffer with 30mM His-200mM sucrose, pH 6 buffer. The 17c10-based ADC was reformulated with 10mM acetate, 1% sorbitol, 3% arginine, pH 5.0 buffer to avoid solubility issues observed during coupling optimization steps. A final sterile filtration (MilliporeMillex-GV Filter, 0.22 µm, PVDF, 13 mm (Ref#SLGVR13SL, lot R0DB27708)) was performed under a microbiological safety cabinet.

For Exatecan-linker payload 1 (EXA1) DAR4 conjugation, Isotypic control and anti-huASCT2 17c10 antibodies (PBS1X, 1mM EDTA, pH7.4 at 8.4 and 5.3 mg/mL, respectively) were reduced by adding 3 antibody molar equivalents of TCEP (Tris(2-carboxyethyl)phosphine) for 2h at +37°C with gentle stirring to allow partial reduction of interchain disulfide bonds. Conjugation was performed by addition of 5.5 antibody molar equivalents of Exatecan-linker payload 1 and incubation for 1h at room temperature under gentle stirring. Residual drug (~1.5 equivalents of drug) was eliminated by diafiltration and formulated in 10 mM acetate, 250 mM sucrose, pH 5.0 buffer. DAR evaluation was performed by LCMS in DTT reduced conditions on an Aeris 3.6 µm Widepore XB-C18 column (200 Å, 150 × 2.1 mm, #00F-4482-AN, Phenomenex) and was estimated to be 4.19 for isotypic control and 3.41 for anti-huASCT2 antibody. Quenching was then performed using 4 drug molar equivalents of N-acetyl cysteine (NAC) for 30min at RT. A final sterile filtration (MilliporeMillex-GV Filter, 0.22 µm, PVDF, 13 mm (Ref#SLGVR13SL, lot R0DB27708)) was performed under a microbiological safety cabinet.

The cysteine reactive linker-exatecan compound 2 (EXA2) was conjugated to cysteine residues of anti-huASCT2 17c10 and isotypic control. In brief, mAbs in PBS 1X, 1 mM EDTA were reduced with 14 molar equivalents of TCEP for 2 hours at 37°C, after which the buffer was exchanged (Amicon ultra 30 kDa) to 100 mM KPO₄, 1 mM EDTA pH 7.4. Twelve molar equivalents of the cysteine reactive linker-exatecan compound 2 were used for conjugation with reactive cysteines for 35 min at room temperature. The exchange buffer was performed in 20 mM His pH 6.0 before filtration 0.22 µM filter. The drug-antibody ratio (DAR) according to LC-MS analysis was 7.89 toxins per conjugated mAb. As determined by SEC-HPLC, for all ADC generated less than 5% material was aggregated.

### Example 3 - In vitro cytotoxicity

Target cells were seeded in triplicates into flat-bottom 384-wells plate (3,000 cells per well for WiDr cells, 400 cells for HCT116 cells). Cells were incubated for 3 to 4 hours at 37°C before addition of an equivalent volume of 2X concentrated ADC or free drug-linker dose range (see table 1 for details). Cells were then incubated into a live cell-imaging instrument (Incucyte^{®}) for 6 to 8 days and their confluency was monitored all along the culture. The resulting proliferation curves were analyzed using GraphPad Prism 7 software.

**Table 1: In vitro cytotoxicity assays summary**

| | HCT116 | WiDr |
|---|---|---|
| 17c10_MC-Dxd | 5 to 2×10⁻⁵ µg/mL | 135 to 6.9×10⁻³ g/mL |
| ICT_MC-Dxd (Isotype control) | 5 to 2×10⁻⁵ µg/mL | 135 to 6.9×10⁻³ g/mL |
| 17c10_βGlu-EXA-2 | 80 to 3.05×10⁻⁴ (µg/mL) | 40 to 1.53×10⁻⁴ (µg/mL) |
| ICT_βGlu-EXA-2 | 80 to 3.05×10⁻⁴ (µg/mL) | 40 to 1.53×10⁻⁴ (µg/mL) |
| MC_Dxd | 9 to 4.6×10⁻⁴ µg/mL | 9 to 4.6×10⁻⁴ µg/mL |
| βGlu-EXA-2 | 2 to 7.62×10⁻⁶ g/mL | 30 to 1.14×10⁻⁴ g/mL |

### Example 4 - ASCT2 expression profile

100,000 cells were seeded per well in 100 µL FACS buffer (PBS1X, 2 % BSA, 2mM EDTA) and incubated with either anti-ASCT2 antibodies or isotypic control (10 µg/mL) for 45min at +4°C. After 3 washings with 150 µL FACS buffer, cells were incubated with Goat anti-human IgG-F(ab)'2-PE secondary antibody for 30min at +4°C, then washed twice. They were finally resuspended in 100 µL FACS buffer containing SYTOX^{®} Blue Dead Cell Stain viability marker (1/10000, Molecular probe # S34857) before analysis on a CytoFLEX flow cytometer (Beckman Coulter). The resulting data were analyzed using FlowJo (BD Biosciences) and GraphPad Prism 7 software.

### Example 5 - In vivo experiments

WiDr cells were cultured in Eagle's minimum essential medium supplemented with 10% FCS and sub-confluent cultures were split every 2 or 3 days. HCT116 cells were cultivated in RPMI supplemented with 10 % FCS, 1 % L-Glutamine 200 mM, 1 % Non-Essential Amino Acids solution and sub-confluent cultures were split every 2 or 3 days. FaDu cells were cultivated in DMEM supplemented with 10 % FCS and sub-confluent cultures were split every 2 or 3 days. NMRI nude female mice were obtained from Janvier Laboratory. Eight-weeks old animals were subcutaneously xenografted with the WiDr cells (2.5 × 10⁶) cells, HCT116 cells (2 × 10⁶) or FaDu cells (5 × 10⁶). WiDr and HCT116 cells were embedded in Matrigel. Treatment with ADC was performed as mentioned in the respective experiments. Tumor volumes (n=10/group) were monitored with a caliper twice a week thereafter and values were reported with the following formula (Length × Width² × (3.14/6).

### References

1. Lund P, Williamson DH. INTER-TISSUE NITROGEN FLUXES. British Medical Bulletin 1985; 41:251-6
2. Hanahan D, Weinberg RA. Hallmarks of cancer: the next generation. Cell 2011; 144:646-74
3. Jin L, Alesi GN, Kang S. Glutaminolysis as a target for cancer therapy. Oncogene 2016; 35:3619-25
4. Hensley CT, Wasti AT, DeBerardinis RJ. Glutamine and cancer: cell biology, physiology, and clinical opportunities. The Journal of Clinical Investigation 2013;123:3678-84
5. Osanai-Sasakawa A, Hosomi K, Sumitomo Y, Takizawa T, Tomura-Suruki S, Imaizumi M, et al. An anti-ASCT2 monoclonal antibody suppresses gastric cancer growth by inducing oxidative stress and antibody dependent cellular toxicity in preclinical models. Am J Cancer Res 2018; 8:1499-513
6. Scalise M, Pochini L, Console L, Losso MA, Indiveri C. The Human SLC1A5 (ASCT2) Amino Acid Transporter: From Function to Structure and Role in Cell Biology. Front Cell Dev Biol 2018; 6:96
7. Cormerais Y, Massard PA, Vucetic M, Giuliano S, Tambutté E, Durivault J, et al. The glutamine transporter ASCT2 (SLC1A5) promotes tumor growth independently of the amino acid transporter LAT1 (SLC7A5). Journal of Biological Chemistry 2018; 293:2877-87
8. Nakaya M, Xiao Y, Zhou X, Chang J-H, Chang M, Cheng X, et al. Inflammatory T Cell Responses Rely on Amino Acid Transporter ASCT2 Facilitation of Glutamine Uptake and mTORC1 Kinase Activation. Immunity 2014; 40:692-705

## Claims

1. A monoclonal antibody or antigen-binding fragment thereof which binds to ASCT2 comprising
(a) a variable heavy chain (VH) region comprising complementarity-determining regions (CDRs) CDR-H1, CDR-H2 and CDR-H3, wherein
(i) the CDR-H1 comprises an amino acid sequence according to SEQ ID NO:1, or an amino acid sequence derived from such sequence comprising a substitution, particularly a conservative substitution of 1 or 2 amino acids,
(ii) the CDR-H2 comprises an amino acid sequence according to SEQ ID NO:2, or an amino acid sequence derived from such sequence comprising a substitution, particularly a conservative substitution of 1 or 2 amino acids,
(iii) the CDR-H3 comprises an amino acid sequence according to SEQ ID NO:3, or an amino acid sequence derived from such sequences comprising a substitution, particularly a conservative substitution of 1 or 2 amino acids,
and optionally
(b) a variable light chain (VL) region, particularly a VL region comprising complementarity-determining regions (CDRs) CDR-L1, CDR-L2 and CDR-L3, wherein
(i) the CDR-L1 comprises an amino acid sequence according to SEQ ID NO:5, or an amino acid sequence derived from such sequences comprising a substitution, particularly a conservative substitution of 1 or 2 amino acids,
(ii) the CDR-L2 comprises an amino acid sequence according to SEQ ID NO:6, or an amino acid sequence derived from such sequences comprising a substitution, particularly a conservative substitution of 1 or 2 amino acids,
(iii) the CDR-L3 comprises an amino acid sequence according to SEQ ID NO:7, or an amino acid sequence derived from such sequences comprising a substitution, particularly a conservative substitution of 1 or 2 amino acids.

2. The monoclonal antibody or antigen-binding fragment thereof according to claim 1, comprising
(a) a variable heavy chain (VH) region comprising complementarity-determining regions (CDRs) CDR-H1, CDR-H2 and CDR-H3, wherein
(i) the CDR-H1 comprises an amino acid sequence according to SEQ ID NO:1,
(ii) the CDR-H2 comprises an amino acid sequence according to SEQ ID NO:2,
(iii) the CDR-H3 comprises an amino acid sequence according to SEQ ID NO:3,
and
(b) a variable light chain (VL) region comprising complementarity-determining regions (CDRs) CDR-L1, CDR-L2 and CDR-L3, wherein
(i) the CDR-L1 comprises an amino acid sequence according to SEQ ID NO:5,
(ii) the CDR-L2 comprises an amino acid sequence according to SEQ ID NO:6,
(iii) the CDR-L3 comprises an amino acid sequence according to SEQ ID NO:7.

3. The antibody or antigen-binding fragment of claim 1 or 2 comprising
(a) a VH region comprising an amino acid sequence according to SEQ ID NO:4, or an amino acid sequence having an identity thereof of at least 85%, at least 90%, at least 95% or at least 99%, and optionally
(b) a VL region, particularly a VL region comprising an amino acid sequence according to SEQ ID NO:8, or an amino sequence having an identity thereof of at least 85%, at least 90%, at least 95% or at least 99%.

4. The antibody or antigen-binding fragment of any of the preceding claims comprising
(a) a VH region comprising an amino acid sequence according to SEQ ID NO:4, and
(b) a VL region comprising an amino acid sequence according to SEQ ID NO:8.

5. The antibody or antigen-binding fragment of any one of claims 1-4, which is a chimeric antibody, a multispecific antibody, in particular a bispecific antibody, a human antibody, a humanized antibody or an antigen-binding fragment thereof.

6. The antibody or antigen-binding fragment of any one of claims 1-5, which is an antibody of class IgG, e.g. of subclass IgG1, IgG2, IgG3 of IgG4, of class IgM, of class IgA or an antigen-binding fragment thereof, or which is a single-chain antibody, or an antibody Fv fragment.

7. The antibody or antigen-binding fragment of any of claims 1-6 comprising a labeling group and/or an effector group being coupled to the antibody or antigen- binding fragment.

8. The antibody or antigen-binding fragment of claim 7, wherein the labeling group is a dye, a paramagnetic, radioactive or fluorogenic group that is detectable upon imaging.

9. The antibody or antigen-binding fragment of claim 7, wherein the effector group is a therapeutic group, in particular a cytotoxic agent, especially a topoisomerase I inhibitor.

10. An antibody-drug conjugate comprising a monoclonal antibody or an antigen-binding fragment thereof according to any of claims 1-9 and a drug, wherein the drug is preferably a topoisomerase I inhibitor, particularly exatecan or deruxtecan.

11. The antibody-drug conjugate according to claim 10, which has a molar drug-antibody/antibody fragment ratio (DAR) of about 2:1 to about 16:1, particularly of about 4:1.

12. The antibody-drug conjugate according to claim 10 or 11, wherein the drug is conjugated to a reactive amino acid residue on the antibody, e.g., an amino acid residue having a side chain comprising an amino, hydroxy or thiol group, or a reactive group in the antibody glycan structure and wherein the drug is particularly conjugated to a reactive thiol group in the side chain of a cysteine residue on the antibody or antigen-binding fragment thereof.

13. The antibody-drug conjugate according to any of claims 10-12, wherein the drug is conjugated to the antibody or antigen-binding fragment thereof via a linker, particularly wherein the linker is particularly a cleavable linker, and/or wherein the linker is particularly a hydrophilic polysarcosine linker, a hydrophilic linker comprising at least one ethylene glycol unit, a linker comprising a highly polar spacer, or an oligopeptide linker.

14. A nucleic acid, in particular a vector, coding for one or more of the antibodies or antigen-binding fragments thereof according to anyone claims 1-9, or for at least one VL and/or one VH of any one of the antibodies of claims 1-9.

15. A pharmaceutical composition comprising an antibody or antigen-binding fragment thereof according to any of claims 1-9 and/or an antibody-drug conjugate according to any of claims 10-13.
